# FASCICULE DE BREVET EUROPEEN

(11) **EP 0 967 250 B1**
(45) Date de publication et mention de la délivrance du brevet: **10.12.2003**
(21) Numéro de dépôt: 99401304.3
(22) Date de dépôt: 01.06.1999
(51) Int. Cl.: C08L 83/06, C08L 83/12, A61K 7/48, A61K 7/027, A61K 7/021, A61K 7/032

(54) **Composition anhydre, utilisation en cosmétique, pharmacie ou hygiène**
Wasserfreie Zusammensetzung, ihre Verwendung in der Kosmetik, Pharmazie oder Körperhygiene
Anhydrous composition, its use in cosmetics, pharmaceutics or personal hygiene

(30) Priorité: 25.06.1998 FR 9808086
(43) Date de publication de la demande: 29.12.1999
(73) Titulaire: L'OREAL, 75008 Paris (FR)
(72) Inventeur: Lemann, Patricia, 94000 Creteil (FR); Collette, Annick, 94600 Choisy Le Roi (FR); Bara, Isabelle, 75013 Paris (FR)
(74) Mandataire: Kromer, Christophe

(56) Documents cités:
- EP-A- 0 548 694
- EP-A- 0 704 205
- WO-A-97/16157
- CA-A- 2 223 742

## Description

La présente invention a pour objet des compositions cosmétiques anhydres à usage cosmétique ou pharmaceutique.

Les compositions de maquillage contenant une phase grasse sont couramment utilisées en cosmétique en raison de leur bonne adhérence à l'épiderme, leur sensation de confort, leur capacité protectrice et leur capacité à former un film imperméable à l'eau. Les produits anhydres de maquillage se présentent en général sous forme solide compacte ou bien sous forme de crème. Ils peuvent également se présenter sous la forme d'un gel fluide.

Ces compositions peuvent constituer des produits de soin de la peau, y compris le cuir chevelu, et/ou des produits de maquillage de la peau, des muqueuses (lèvres ou intérieurs des paupières), des semi-muqueuses (lèvres), des fibres kératiniques (cheveux, cils, ongles) ou encore des produits de maquillage du corps.

Toutefois, un des inconvénients de ce type de produit est qu'ils ne sont pas stables.

De plus, ces compositions, lorsqu'elles sont appliquées sur la peau, les muqueuses ou les semi-muqueuses, présentent l'inconvénient de transférer. On entend par là que la composition est susceptible de se déposer, au moins en partie, sur certains supports avec lesquels elle est mise en contact, tels que, par exemple, un verre, un vêtement ou la peau.

En se déposant, ladite composition laisse une trace sur ledit support. Il s'en suit donc une persistance médiocre de la composition sur la peau ou les muqueuses, d'où la nécessité de renouveler régulièrement son application.

Par ailleurs, l'apparition de traces inacceptables sur certains vêtements et notamment sur les cols de chemisier peut écarter certaines femmes de l'utilisation de ce type de maquillage.

Un autre inconvénient de ces compositions réside dans le problème de migration. On a en effet constaté que certaines compositions avaient tendance à se propager à l'intérieur des ridules et/ou des rides de la peau, dans le cas des fonds de teint ; dans les ridules qui entourent les lèvres, dans le cas des rouges à lèvres ; dans les plis de la paupière, dans le cas des fards à paupières. On a également constaté, dans le cas notamment des fards à paupières, l'apparition de stries dans le maquillage, générées par les mouvements des paupières.

Tous ces phénomènes engendrent un effet inesthétique que l'on souhaite bien évidemment éviter.

Dans le but de diminuer ces phénomènes, il a été proposé dans WO 97/16157 d'associer à un solvant volatil un tensio-actif polymérique de type organosiloxane ayant au moins un radical hydrophile et au moins un radical lipophile.

Toutefois, si l'on désire introduire des pigments dans ces produits, on se trouve souvent confronté à un problème d'homogénéité des pigments.

Dans tous les cas, les compositions obtenues présentent des défauts tant au niveau de la stabilité qu'au niveau de l'homogénéité de la dispersion des pigments.

Le but de la présente invention est de fournir une composition anhydre qui présente une bonne stabilité, qui transfère peu et qui présente une très bonne dispersion des pigments et ainsi des propriétés cosmétiques améliorées.

Il a maintenant été découvert de façon inattendue et surprenante que par l'emploi d'un tensio-actif particulier, il était possible d'obtenir des compositions anhydres ayant non seulement une bonne stabilité dans le temps mais encore vis-à-vis des variations de température et présentant de plus d'excellentes propriétés cosmétiques, en particulier une dispersion homogène des pigments.

La présente invention a donc pour objet une composition selon la revendication 1.

L'invention porte également sur un procédé de traitement non thérapeutique de la peau et/ou du cuir chevelu, notamment un procédé de maquillage, consistant à appliquer sur la peau ou les muqueuses et/ou sur le cuir chevelu une composition telle que définie ci-dessus.

La composition anhydre selon l'invention est particulièrement stable.

La composition anhydre selon l'invention est particulièrement homogène. Elle permet un maquillage uniforme et homogène.

La composition anhydre selon l'invention présente en outre une bonne résistance au transfert. De plus, appliquée sur la peau, elle présente l'avantage de ne pas migrer dans les plis de la peau et/ou les rides du visage.

On a constaté que la composition utilisée selon l'invention s'applique et s'étale facilement de façon homogène, sans laisser de sensation de gras et présente de bonnes propriétés cosmétiques. Le film obtenu présente également une texture légère et reste confortable à porter tout au long de la journée.

La composition selon l'invention possède par ailleurs de bonnes qualités sensorielles notamment une grande facilité d'application, du confort, de la douceur, une bonne matité et une bonne couvrance, de l'uniformité et de la tenue.

Les compositions de l'invention sont des compositions anhydres. Par composition anhydre, on entend une composition comprenant moins de 5 % d'eau en poids, par rapport au poids total de la composition, de préférence entre 1 % et 2 % d'eau. Plus préférentiellement encore, la composition ne comprend pas d'eau du tout. De préférence, les compositions de l'invention sont exemptes d'alcools polyvalents, c'est-à-dire d'alcools comprenant au moins deux groupes OH comme le propylène glycol, le butylène glycol, la glycérine, le sorbitol.

Dans tout ce qui suit ou qui précède, on entend désigner par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀ et en particulier méthyle, les radicaux fluoroalkyles, les radicaux aryles et en particulier phényle.

Ainsi, la silicone oxyalkylénée substituée en α-ω utilisable pour la composition selon l'invention est un polymère organosilicié tel que défini ci-dessus, à structure linéaire, substitué aux deux extrémités de la chaîne principale par des groupements oxyalkylène reliés aux atomes de Si par l'intermédiaire d'un groupement hydrocarboné. De préférence, la chaîne principale ne comprend pas de groupement oxyalkylène pendant.

De préférence, la silicone oxyalkylénée substituée en α-ω répond à la formule générale (I) suivante : 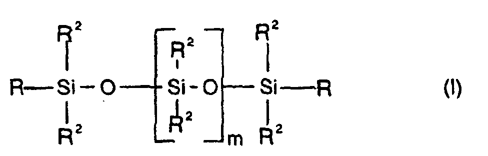 dans laquelle : R = (CH₂)ₚ-O-(C₂H₄O)ₓ (C₃H₆O)_{Y}R¹
où :
- R¹ représente H, CH₃ ou CH₂CH₃,
- p est un entier allant de 1 à 5, x varie de 1 à 100, y varie de 0 à 50,
- les unités (C₂H₄O) et (C₃H₆O) pouvant être réparties de façon aléatoire ou par blocs,

- les radicaux R² représentent un radical alkyle en C1-C3 ou un radical phényle,
- 5 ≤ m ≤ 300.

De préférence, la silicone oxyalkylénée substituée en α-ω utilisée selon la présente invention répond à la formule générale (I) pour laquelle tous les radicaux R² sont des radicaux méthyles et :
- p va de 2 à 4,
- x va de 3 à 100,
- m va de 50 à 200.

De préférence encore, le poids moléculaire moyen de R va de 800 à 2600.

De préférence, le rapport en poids des unités C₂H₄O par rapport aux unités C₃H₆O va de 100:10 à 20:80.

De préférence, ce rapport est d'environ 42/58.

De préférence encore, R¹ est le groupe méthyle.

De façon plus préférentielle encore, la composition selon l'invention comprend la silicone oxyalkylénée en α-ω de formule suivante : 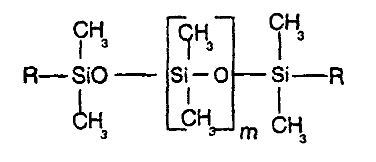 dans laquelle :
- m=100,
- R = (CH₂)₃-O-(C₂H₄O)ₓ (C₃H₆O)_{y}-CH₃, où x va de 3 à 100, y va de 1 à 50, le rapport en poids du nombre de C₂H₄O sur le nombre de C₃H₆O étant d'environ 42/58, le poids moléculaire moyen de R allant de 800 à 1500.

La silicone oxyalkylénée substituée en α-ω telle que définie ci-dessus est utilisée selon l'invention en une proportion allant de 0,1 à 20 % de préférence allant de 0,1 à 10 % en poids par rapport au poids total de la composition.

Parmi. les produits du commerce pouvant contenir tout ou partie des silicones oxyalkylénées substituées en α-ω utilisables selon l'invention comme émulsionnant on peut citer notamment ceux vendus sous les dénominations de "Abil EM 97" par la Société Goldschmidt, ou encore de "KF 6009", "X22-4350", "X22-4349" ou "KF 6008" par la Société Shin Etsu.

Les compositions selon l'invention comprennent au moins un pigment.

Les pigments peuvent être présents dans la composition à une teneur allant de 0,1 à 20 % en poids, par rapport au poids total de la composition, et de préférence à raison de 2-15 %. Ils peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique, les nacres telles que le mica recouvert d'oxyde de titane, d'oxyde de fer, de pigment naturel ou d'oxychlorure de bismuth ainsi que le mica titane coloré. Parmi les pigments organiques, on peut citer le noir de carbone, et les laques de baryum, strontium, calcium, aluminium. Les pigments peuvent également présenter une surface hydrophobe ou peuvent être traités de manière à rendre leur surface hydrophobe; ce traitement peut être effectué selon les méthodes connues de l'homme du métier; les pigments peuvent notamment être enrobés par des composés siliconés tels que des PDMS et/ou par des polymères, notamment des polyéthylènes et/ou des acides aminés.

Parmi les pigments enrobés, on peut citer notamment les pigments vendus sous la dénomination de "Covasil" par la Société WACKER (pigments au triisostéaroyltitanate).

Les pigments ainsi enrobés peuvent être incorporés dans la composition selon l'invention en une proportion comprise entre 0,1 et 15 % en poids par rapport au poids total de la composition.

La composition selon l'invention comprend au moins un solvant volatil. Par solvant volatil, on entend un composé qui s'évapore à température ambiante (T = 25°C). De préférence, le solvant volatil a une vicosité allant de 0,5 à 25 centistokes à 25°C. Les solvants volatils utilisables selon la présente invention peuvent par exemple être des silicones linéaires ou cycliques, c'est-à-dire des polydiorganosiloxanes linéaires ou cycliques, éventuellement fonctionnalisés, ou encore des paraffines hydrocarbonées ou leurs mélanges.

Les polydiorganosiloxanes linéaires éventuellement fonctionnalisés utilisables selon l'invention répondent à la formule générale suivante : 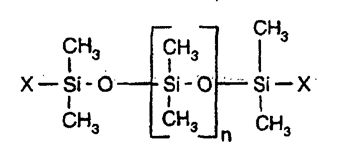 dans laquelle :
- X est CH₃ ou OH, et
- n est un entier allant de 0 à 2000.

Parmi ceux-ci, on citera notamment les produits vendus sous la dénomination de "AK" par la Société WACKER, "SF" par la Société GENERAL ELECTRIC et "ABIL" par la Société GOLDSCHMIDT, tel que le produit "Abil 10".

Comme polydiorganosiloxanes cycliques selon l'invention, on peut utiliser, seuls ou en mélange, des cyclométhicones de formule : 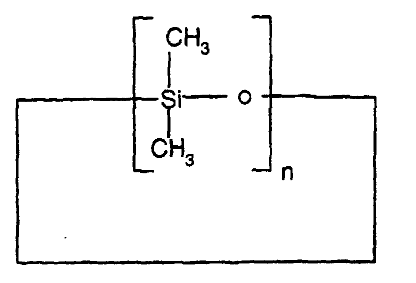 dans laquelle :
- n est un nombre entier de 3 à 8.

Parmi les cyclométhicones particulièrement préférées, on citera le cyclotétradiméthylsiloxane (n = 4), le cyclopentadiméthylsiloxane (n = 5), et le cyclohexadiméthylsiloxane (n = 6).

On peut notamment utiliser les produits vendus sous les dénominations de "DC Fluid 244", "DC Fluid 245", "DC FLuid 344" et "DC Fluid 345" par la Société Dow Corning.

D'autres cyclométhicones utilisables selon l'invention sont celles vendues sous les dénominations de "Abil K4" par la Société GOLDSCHMIDT ; sous les dénominations de "Silbione 70045 V2" et de "Silbione Huile 70045 V5" par la Société RHONE POULENC ; ainsi que sous les dénominations de "Volatil Silicone 7158" et de "Volatil Silicone 7207" par la Société UNION CARBIDE.

Les solvants volatils peuvent aussi être des paraffines à chaînes hydrocarbonées droites ou ramifiées ayant de 8 à 40 atomes de carbone, de préférence de 10 à 20 atomes de carbone. Ces solvants sont par exemple les décane, dodécane, tétradécane, tridécane, les isoparaffines en C8-C20 comme par exemple celles décrites dans US 3, 439, 088 et US 3, 818, 105. Les paraffines préférées selon la présente invention sont par exemple celles ayant un poids moléculaire allant de 160 à 180. De préférence également, elles ont un point d'ébullition allant de 105 à 320 °C. De préférence encore, les paraffines ont une viscosité inférieure ou égale à 20 cst (centistokes) à 25 °C. De telles paraffines sont vendues par exemple par EXXON sous le nom de marque "ISOPARS".

Le solvant volatil utilisé dans les compositions selon l'invention est généralement présent dans les compositions de l'invention à une teneur allant de 0,5 à 80 %, en poids, par rapport au poids total de la composition, de préférence à une teneur allant de 5 à 80%.

De préférence, on utilise les huiles de silicone volatiles, et de préference encore les cyclométhicones.

Les compositions selon l'invention peuvent également comprendre des corps gras non volatils.

Parmi les corps gras non volatils, on peut citer les huiles non volatiles, les corps gras pâteux, les gommes et les cires végétales, minérales, animales et/ou synthétiques, ces derniers comprenant les corps gras siliconés.

Les compositions selon l'invention peuvent également comprendre des huiles non volatiles. De préférence, ces huiles ont une viscosité allant de 0,5 à 1000 000 centistokes, de préférence encore, allant de 25 à 600 000 centistokes à 25 °C. Des exemples de telles huiles sont les huiles essentielles, les esters, les esters de glycérol d'acides gras, les acides gras, les alcools gras et leurs mélanges. Des exemples d'esters convenant aux compositions selon l'invention sont les acétylglycérides, les octanoates, décanoates, ricinoléates d'alcools ou de polyalcools ; l'huile de ricin, la lanoline et ses dérivés, l'huile de palme, l'huile d'olive, l'huile de soja, les huiles de germes de céréales.

Des exemples d'huiles convenant également à la présente invention sont des paraffines non volatiles comme le polyisobutène, les huiles minérales, le polydécène, le squalane, le pétrolatum.

De préférence, ces huiles sont présentes dans la composition selon l'invention à une teneur allant de 0,1 à 80% en poids, par rapport au poids total de la composition, de préférence encore, de 0,1 à 50%.

Les compositions selon l'invention peuvent également comprendre des cires.

Comme cires utilisables dans l'invention, on peut citer les cires d'origine animale comme la lanoline, la cire d'abeilles, le spermaceti, les dérivés de la lanoline tels que les alcools de lanoline, la lanoline hydrogénée, hydroxylée ou acétylée, les acides gras de la lanoline et l'alcool de lanoline acétylée ; les cires d'origine végétale telles que la cire de Carnauba, de Candelilla, de kapok, d'Ouricury, de riz, de jojoba hydrogénée, d'Alfa, du Japon ou les cires de fibres de liège ou de canne à sucre ou encore le beurre de cacao ; les cires minérales par exemple de paraffine, de montan, de lignite, de pétrolatum, de vaseline ou les cires microcristallines, la cérésine, l'ozokérite ; les cires synthétiques comme les cires de polyéthylène, les cires obtenues par synthèse de Fischer-Tropsch et les esters linéaires résultant de la réaction d'un acide carboxylique saturé en C₁₀ à C₄₀ et d'un alcool saturé en C₁₀ à C₄₀ comme le myristate de myristyle. On peut aussi utiliser l'alcool cétylique, l'alcool stéarylique, les lanolates ou stéarates de calcium, l'huile de ricin, de palme, de coco, de tournesol ou de coprah hydrogénée.

Les cires peuvent être présentes dans les compositions selon l'invention généralement à une teneur pouvant aller jusqu'à 40% en poids, par rapport au poids total de la composition, et de préférence encore jusqu'à 20%.

On peut définir les composés gras pâteux à l'aide d'au moins une des propriétés physico-chimiques suivantes :
- une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 60 Hz,
- un point de fusion de 25-70°C, de préférence 25-55°C.

Les compositions de l'invention peuvent également comprendre des alkyl, alcoxy ou phényl-diméthicones tels que, par exemple le produit vendu sous la dénomination de "Abil wax 2440" par la Société GOLDSCHMIDT.

Les compositions selon l'invention peuvent également comprendre des résines de silicone comprenant une combinaison des unités R₃SiO_{1/2} , R₂SiO_{2/2} , RSiO_{3/2} et SiO_{4/2}, R représentant un groupe alkyle ayant de 1 à 6 atomes de carbone ou un groupe phényle.

Parmi les corps gras siliconés, on peut citer les polyalkyl(C₁-C₂₀)siloxanes, les huiles de silicone phénylées comme par exemple la phényl triméthicone vendue sous la dénomination commerciale "Abil AV 1000" par Goldschmidt, ainsi que les gommes de silicones et les cires de silicone.

Les gommes de silicone peuvent répondre à la formule : 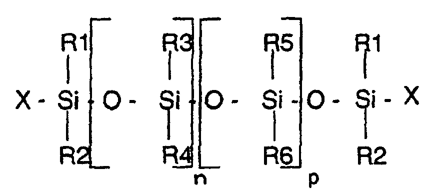 dans laquelle:
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle et notamment phényle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.
De manière générale, n et p peuvent prendre des valeurs de 0 à 5000, de préférence de 0 à 3000.

Comme gomme de silicone utilisable selon l'invention, on peut citer celles pour lesquelles :
- les substituants R1 à R6 et X représentent un groupement méthyle, p = 0 et n = 2700, comme celle vendue sous la dénomination SE30 par la société General Electric,
- les substituants R1 à R6 et X représentent un groupement méthyle, p = 0 et n = 2300, comme celle vendue sous la dénomination AK 500000 par la société Wacker,
- les substituants R1 à R6 représentent un groupement méthyle, le substituant X représente un groupement hydroxyle, p = 0 et n = 2700, en solution à 13 % dans du cyclopentasiloxane, comme celle vendue sous la dénomination Q2-1401 par la société Dow Corning,
- les substituants R1 à R6 représentent un groupement méthyle, le substituant X représente un groupement hydroxyle, p = 0 et n = 2700, en solution à 13% dans du polydiméthylsiloxane, comme celle vendue sous la dénomination Q2-1403 par la société Dow Corning,
- les substituants R1, R2, R5, R6 et X représentent un groupement méthyle, les substituants R3 et R4 représentent un groupement phényle tel que le poids moléculaire du composé soit de 600 000, comme celle vendue sous les dénominations "761" ou "MIRASIL C-DPDM" par la société Rhône-Poulenc.

Ces corps gras peuvent en particulier être choisis de manière variée par l'homme du métier afin de préparer une composition ayant les propriétés souhaitées, par exemple en consistance ou en texture. Ils sont de préférence utilisés à une teneur inférieure ou égale à 7 % en poids par rapport au poids total de l'émulsion, afin de conserver les propriétés avantageuses de l'émulsion utilisée selon l'invention.

Parmi les autres adjuvants liposolubles que l'on peut incorporer à la composition, on peut citer les filtres U.V. lipophiles, les vitamines lipophiles, les antioxydants et les parfums, les céramides.

La composition selon l'invention peut également comprendre une phase particulaire qui peut comprendre, outre les pigments déjà cités ci-dessus, des nacres et/ou des charges habituellement utilisés dans les compositions cosmétiques.

Les charges, qui peuvent être présentes dans la composition à raison de 0-20 % en poids, par rapport au poids total de la composition, de préférence 0-10 %, peuvent être minérales ou de synthèse, lamellaires ou non lamellaires. On peut citer le talc, le mica, la silice, le kaolin, le Téflon, l'amidon, la nacre naturelle, le nitrure de bore, les microsphères telles que l'Expancel (Nobel Industrie), les microéponges telles que le polytrap (Dow Corning). De préférence, on utilise des charges sphériques dont la taille est inférieure à 25 µm telles que les poudres de polyéthylène, les poudres de Nylon, les microbilles de résine de silicone (Tospearls de Toshiba), les microsphères de silice, ces charges pouvant contribuer à améliorer les propriétés non transfert des compositions de l'invention.

La composition selon l'invention peut comprendre en outre un milieu cosmétiquement, pharmaceutiquement ou hygiéniquement acceptable. Elle peut comprendre alors tout additif usuellement utilisé dans le domaine cosmétique, pharmaceutique ou hygiénique, tels que des antioxydants, des colorants, des parfums, des huiles essentielles, des conservateurs, des actifs cosmétiques, des hydratants, des vitamines, des sphingolipides, des polymères liposolubles notamment hydrocarbonés, tels que le polybutène, les polyalkylènes, les polyacrylates et les polymères siliconés compatibles avec les corps gras.

Ces additifs peuvent être présents dans la composition à raison de 0-10% en poids.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous la forme d'un produit cosmétique et notamment sous la forme d'un produit de soin pour le corps et/ou le visage et/ou le cuir chevelu ou bien encore d'un produit de maquillage, en particulier un fond de teint, un fard à joues ou à paupières, un eye-liner, un mascara ou un rouge à lèvres.

Elles peuvent également se présenter sous forme non colorée, contenant éventuellement des actifs cosmétiques.

Les compositions selon l'invention peuvent se présenter sous la forme d'un gel fluide ou d'un stick.

De préférence, les compositions selon l'invention se présentent sous une forme fluide et ont une viscosité Brookfield, mesurée sur le modèle LVDV, avec une aiguille de 3, à une vitesse de 60 trs/min et à environ 25°C, allant de 8 poises à 50 poises.

L'invention porte encore sur l'utilisation d'une silicone oxyalkylénée substituée en α-ω telle que définie ci-dessus dans une composition anhydre comprenant des pigments et un solvant volatil dans le but d'améliorer la dispersion desdits pigments dans ladite composition.

L'invention porte également sur l'utilisation d'une silicone oxyalkylénée substituée en α-ω telle que définie ci-dessus dans une composition anhydre comprenant des pigments et un solvant volatil dans le but d'améliorer l'homogénéité de ladite composition.

### EXEMPLE COMPARATIF :

La Demanderesse a réalisé les compositions anhydres suivantes :

| Composition A (conforme à l'invention) : | |
|---|---|
| - cyclométhicone | 97,5 g |
| | |
| - mélange silicone oxyéthylénée oxypropylénée substituée en α-ω/cyclométhicone (85/15) vendu sous la dénomination commerciale "Abil EM 97" par la Société Goldschmidt | 2,5 g |
| | |
| - pigments | 50 g |

| Composition B (comparative) : | |
|---|---|
| - cyclométhicone | 97,5 g |
| | |
| - silicone à groupements alkyle, oxyéthylène et oxypropylène pendants dans un mélange polyglycéryl-4-isostéarate et hexyllaurate vendue sous la dénomination commerciale "Abil WE 09" par la société Goidschmidt | 2,5 g |
| | |
| - pigments | 50 g |

La Demanderesse a ensuite mesuré les viscosités au temps t = 3 minutes des compositions A et B. Ces viscosités ont été mesurées au Brookfield, modèle LVDV, avec une aiguille de 3, à une vitesse de 60 trs/min et à environ 25°C. Les résultats sont regroupés dans le tableau suivant :

| **Composition** | **Viscosité Brookfield** en poises |
|---|---|
| A (conforme à l'invention) | 8,8 |
| B (comparative) | 14 |

Ainsi, la composition anhydre A, pour le même taux de pigment, est beaucoup plus fluide que la composition B. La composition A présente une texture plus homogène que la composition B. Les pigments sont mieux dispersés dans la composition A selon l'invention que dans la composition B.

## Revendications

1. Composition anhydre ne contenant pas d'eau du tout se présentant sous forme de produit de soin de la peau ou de produit de maquillage, la composition comprenant au moins un pigment et au moins un solvant volatil choisi parmi :
a) les polydiorganosiloxanes linéaires de formule générale suivante : 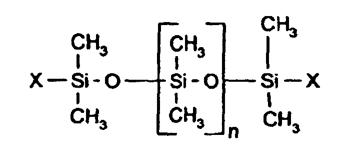 dans laquelle :
- X est CH₃ ou OH, et
- n est un entier allant de 0 à 2000 ;
b) les polydiorganosiloxanes cycliques de formule: 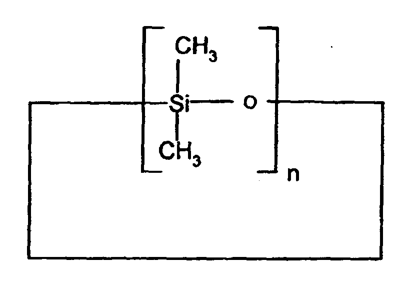 dans laquelle :
- n est un nombre entier de 3 à 8 ;
c) les paraffines à chaînes hydrocarbonées droites ou ramifiées ayant de 8 à 40 atomes de carbone;
**caractérisée par le fait qu'**elle comprend au moins une silicone oxyalkylénée substituée en α-ω

2. Composition selon la revendication 1, **caractérisée par le fait que** la silicone oxyalkylénée substituée en α-ω est un polymère organosilicié à structure linéaire, substitué aux deux extrémités de la chaîne principale par des groupements oxyalkylène reliés aux atomes de Si par l'intermédiaire d'un groupement hydrocarboné.

3. Composition selon la revendication 2, **caractérisée par le fait que** la chaîne principale ne comprend pas de groupement oxyalkylène pendant.

4. Composition selon l'une quelconque des revendications 1 à 3, **caractérisée par le fait que** la silicone oxyalkylénée substituée en α-ω répond à la formule générale (I) suivante : 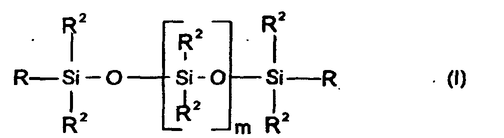 dans laquelle : R = -(CH₂)ₚ-O-(C₂H₄O)ₓ (C₃H₆O)_{Y}R¹
où :
- R¹ représente H, CH₃ ou CH₂CH₃,
- p est un entier allant de 1 à 5, x varie de 1 à 100, y varie de 0 à 50,
- les unités (C₂H₄O) et (C₃H₆O) pouvant être réparties de façon aléatoire ou par blocs,
- les radicaux R² représentent un radical alkyle en C1-C3 ou un radical phényle,
- 5 ≤ m ≤ 300.

5. Composition selon la revendication 4, **caractérisée par le fait que** la silicone oxyalkylénée substituée en α-ω utilisée selon la présente invention répond à la formule générale (1) pour laquelle tous les radicaux R² sont des radicaux méthyles et :
- p va de 2 à 4,
- x va de 3 à 100,
- m va de 50 à 200.

6. Composition selon la revendication 4 ou 5, **caractérisée par le fait que** le poids moléculaire moyen de R va de 800 à 2600.

7. Composition selon l'une quelconque des revendications 4 à 6, **caractérisée par le fait que** le rapport en poids des unités C₂H₄O par rapport aux unités C₃H₆O va de 100:10 à 20:80.

8. Composition selon la revendication 7, **caractérisée par le fait que** ce rapport est d'environ 42/58.

9. Composition selon l'une quelconque des revendications 4 à 8, **caractérisée par le fait qu'**elle comprend la silicone oxyalkylénée substituée en α-ω de formule suivante : 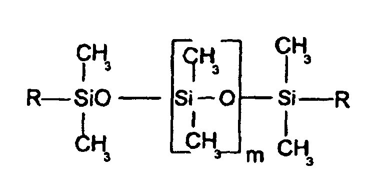 dans laquelle :
- m=100,
- R = -(CH₂)₃-O-(C₂H₄O)ₓ (C₃H₆O)_{y}-CH₃, où x va de 3 à 100, y va de 1 à 50, le rapport en poids du nombre de (C₂H₄O) sur le nombre de (C₃H₆O) étant d'environ 42/58, le poids moléculaire moyen de R allant de 800 à 1500.

10. Composition selon l'une quelconque des revendications 1 à 9, **caractérisée par le fait que** la silicone oxyalkylénée substituée en α-ω est présente dans la composition en une proportion allant de 0,1 à 20% en poids par rapport au poids total de la composition

11. Composition selon la revendication 10, **caractérisée par le fait que** la proportion en silicone oxyalkylénée substituée en α-ω va de 0,1 à 10% en poids par rapport au poids total de la composition.

12. Composition selon l'une quelconque des revendications 1 à 11, **caractérisée par le fait que** les pigments sont choisis parmi les dioxydes de titane, de zirconium ou de cérium, ainsi que les oxydes de zinc, de fer ou de chrome, le bleu ferrique, les nacres telles que le mica recouvert d'oxyde de titane, d'oxyde de fer, de pigment naturel ou d'oxychlorure de bismuth ainsi que le mica titane coloré, le noir de carbone, les laques de baryum, strontium, calcium, aluminium, les pigments enrobés par des composés siliconés tels que des PDMS et/ou par des polymères, notamment des polyéthylènes et/ou des acides aminés.

13. Composition selon l'une quelconque des revendications 1 à 12, **caractérisée par le fait que** les pigments sont présents dans la composition à une teneur allant de 0,1 à 20% en poids, par rapport au poids total de la composition.

14. Composition selon la revendication 13, **caractérisée par le fait que** la teneur en pigments va de 2 à 15% en poids, par rapport au poids total de la composition.

15. Composition selon l'une quelconque des revendications 1 à 14, **caractérisée par le fait que** le solvant volatil a une viscosité allant de 0,5 à 25 centistokes à 25 °C.

16. Composition selon l'une quelconque des revendications 1 à 15, **caractérisée par le fait que** le solvant volatil est choisi parmi les paraffines à chaînes hydrocarbonées droites ou ramifiées ayant de 10 à 20 atomes de carbone.

17. Composition selon l'une quelconque des revendications 1 à 16, **caractérisée par le fait que** le solvant volatil est choisi parmi les décane, dodécane, tétradécane, tridécane, les isoparaffines en C8-C20.

18. Composition selon l'une quelconque des revendications 1 à 16, **caractérisée par le fait que** le solvant volatil est choisi parmi les paraffines à chaînes hydrocarbonées droites ou ramifiées ayant de 10 à 40 atomes de carbone et ayant un poids moléculaire allant de 160 à 180 ou ayant un point d'ébullition allant de 105 à 320 °C.

19. Composition selon l'une quelconque des revendications 1 à 18, **caractérisée par le fait que** le solvant volatil est présent dans la composition en une proportion allant de 0,5% à 80% en poids par rapport au poids total de la composition.

20. Composition selon la revendication 19, **caractérisée par le fait que** la teneur en solvant volatil va de 5 à 80% en poids, par rapport au poids total de la composition.

21. Composition selon l'une quelconque des revendications 1 à 20, **caractérisée par le fait qu'**elle comprend une cire.

22. Composition selon l'une quelconque des revendications 1 à 21, **caractérisée par le fait qu'**elle comprend une charge choisie parmi le talc, le mica, la silice, le kaolin, le Téflon, l'amidon, la nacre naturelle, le nitrure de bore, les microsphères, les microéponges, les poudres de polyéthylène, les poudres de Nylon, les microbilles de résine de silicone, les microsphères de silice.

23. Composition selon l'une quelconque des revendications 1 à 22, **caractérisée par le fait que** le produit de maquillage est un fond de teint, un fard à joues ou à paupières, un eye-liner, un mascara ou un rouge à lèvres.

24. Composition selon l'une quelconque des revendications 1 à 23, **caractérisée par le fait qu'**elle se présente sous la forme d'un gel fluide ou d'un stick.

25. Procédé de traitement non thérapeutique de la peau et/ou du cuir chevelu, notamment un procédé de maquillage, consistant à appliquer sur la peau ou les muqueuses et/ou sur le cuir chevelu une composition telle que définie à l'une quelconque des revendications 1 à 24.

26. Utilisation d'une silicone oxyalkylénée substituée en α-ω telle que définie à l'une quelconque des revendications 1 à 9 dans une composition anhydre ne contenant pas d'eau se présentant sous forme de produit de soin de la peau ou de produit de maquillage comprenant des pigments et un solvant volatil choisi parmi:
a) les polydiorganosiloxanes linéaires de formule générale suivante : 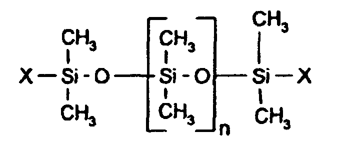 dans laquelle :
- X est CH₃ ou OH, et
- n est un entier allant de 0 à 2000 ;
b) les polydiorganosiloxanes cycliques de formule : 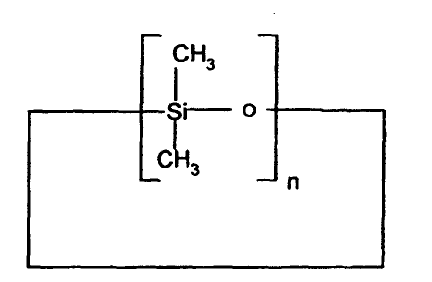 dans laquelle :
- n est un nombre entier de 3 à 8 ;
c) les paraffines à chaînes hydrocarbonées droites ou ramifiées ayant de 8 à 40 atomes de carbone;
dans le but d'améliorer la dispersion desdits pigments dans ladite composition.

27. Utilisation d'une silicone oxyalkylénée substituée en α-ω telle que définie à l'une quelconque des revendications 1 à 9 dans une composition anhydre ne contenant pas d'eau se présentant sous forme de produit de soin de la peau ou de produit de maquillage comprenant des pigments et un solvant volatil choisi parmi :
a) les polydiorganosiloxanes linéaires de formule générale suivante : 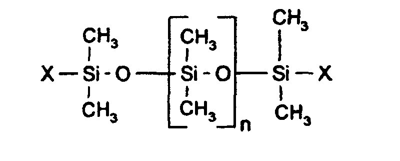 dans laquelle :
- X est CH₃ ou OH, et
- n est un entier allant de 0 à 2000 ;
b) les polydiorganosiloxanes cycliques de formule : 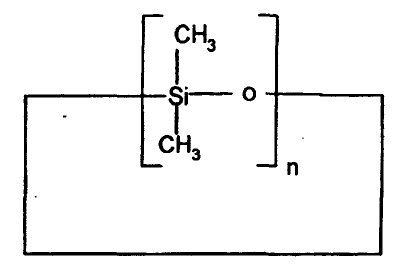 dans laquelle :
- n est un nombre entier de 3 à 8 ;
c) les paraffines à chaînes hydrocarbonées droites ou ramifiées ayant de 8 à 40 atomes de carbone;
dans le but d'améliorer l'homogénéité de ladite composition.

## Claims

1. Anhydrous composition containing no water at all, which is in the form of a skincare product or a makeup product, the composition comprising at least one pigment and at least one volatile solvent chosen from:
a) linear polydiorganosiloxanes having the general formula below: 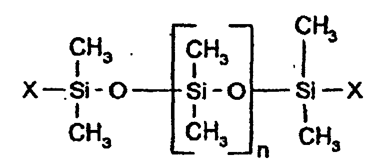 in which:
- X is CH₃ or OH, and
- n is an integer ranging from 0 to 2000;
b) cyclic polydiorganosiloxanes of formula: 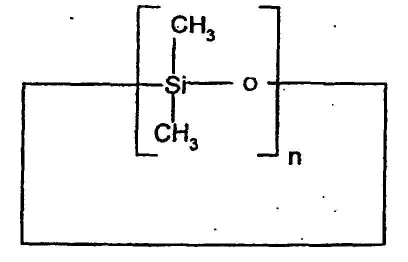 in which:
- n is an integer from 3 to 8;
c) paraffins containing straight or branched hydrocarbon-based chains containing from 8 to 40 carbon atoms;
**characterized in that** it comprises at least one α,ω-substituted oxyalkylenated silicone.

2. Composition according to Claim 1, **characterized in that** the α,ω-substituted oxyalkylenated silicone is an organosilicon polymer of linear structure, substituted at both ends of the main chain with oxyalkylene groups linked to the Si atoms via a hydrocarbon-based group.

3. Composition according to Claim 2, **characterized in that** the main chain does not comprise any pendent oxyalkylene groups.

4. Composition according to any one of Claims 1 to 3, **characterized in that** the α,ω-substituted oxyalkylenated silicone corresponds to the general formula (I) below: 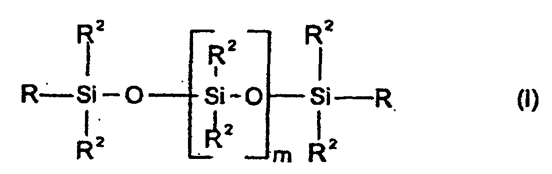 in which: R = -(CH₂)ₚ-O-(C₂H₄O)ₓ (C₃H₆O)_{Y}R¹
in which:
- R¹ represents H, CH₃ or CH₂CH₃,
- p is an integer ranging from 1 to 5, x ranges from 1 to 100 and y ranges from 0 to 50,
- the (C₂H₄O) and (C₃H₆O) units possibly being distributed randomly or in blocks,
- the radicals R² represent a C₁-C₃ alkyl radical or a phenyl radical,
- 5 ≤ m ≤ 300.

5. Composition according to Claim 4, **characterized in that** the α,ω-substituted oxyalkylenated silicone used according to the present invention corresponds to the general formula (I) for which all the radicals R² are methyl radicals, and:
- p ranges from 2 to 4,
- x ranges from 3 to 100,
- m ranges from 50 to 200.

6. Composition according to Claim 4 or 5, **characterized in that** the average molecular weight of R ranges from 800 to 2600.

7. Composition according to any one of Claims 4 to 6, **characterized in that** the weight ratio of the C₂H₄O units relative to the C₃H₆O units ranges from 100:10 to 20:80.

8. Composition according to Claim 7, **characterized in that** this ratio is about 42/58.

9. Composition according to any one of Claims 4 to 8, **characterized in that** it comprises the α,ω-substituted oxyalkylenated silicone having the formula below: 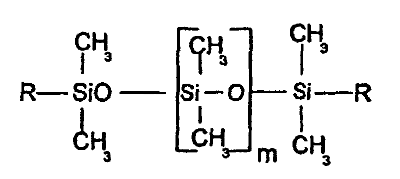 in which:
- m = 100,
- R = (CH₂)₃-O-(C₂H₄O)ₓ (C₃H₆O)_{y}-CH₃, in which x ranges from 3 to 100 and y ranges from 1 to 50, the weight ratio of the number of C₂H₄O to the number of C₃H₆O being about 42/58, the average molecular weight of R ranging from 800 to 1500.

10. Composition according to any one of Claims 1 to 9, **characterized in that** the α,ω-substituted oxyalkylenated silicone is present in the composition in a proportion ranging from 0.1% to 20% by weight relative to the total weight of the composition.

11. Composition according to Claim 10, **characterized in that** the proportion of α,ω-substituted oxyalkylenated silicone ranges from 0.1% to 10% by weight relative to the total weight of the composition.

12. Composition according to any one of Claims 1 to 11, **characterized in that** the pigments are chosen from titanium dioxide, zirconium dioxide and cerium dioxide, and also zinc oxide, iron oxide or chromium oxide, ferric blue, nacres such as mica coated with titanium oxide, with iron oxide, with natural pigment or with bismuthoxychloride, and also coloured titanium mica, carbon black, barium, strontium, calcium or aluminium lakes, and pigments coated with silicone compounds such as PDMSs and/or with polymers, especially polyethylenes and/or amino acids.

13. Composition according to any one of Claims 1 to 12, **characterized in that** the pigments are present in the composition in a content ranging from 0.1% to 20% by weight relative to the total weight of the composition.

14. Composition according to Claim 13, **characterized in that** the pigment content ranges from 2% to 15% by weight relative to the total weight of the composition.

15. Composition according to any one of Claims 1 to 14, **characterized in that** the volatile solvent has a viscosity ranging from 0.5 to 25 centistokes at 25°C.

16. Composition according to any one of Claims 1 to 15, **characterized in that** the volatile solvent is chosen from paraffins containing straight or branched hydrocarbon-based chains containing from 10 to 20 carbon atoms.

17. Composition according to any one of Claims 1 to 16, **characterized in that** the volatile solvent is chosen from decane, dodecane, tetradecane, tridecane and C₈-C₂₀ isoparaffins.

18. Composition according to any one of Claims 1 to 16, **characterized in that** the volatile solvent is chosen from paraffins containing straight or branched hydrocarbon-based chains containing from 10 to 40 carbon atoms and having a molecular weight ranging from 160 to 180 or having a boiling point ranging from 105 to 320°C.

19. Composition according to any one of Claims 1 to 18, **characterized in that** the volatile solvent is present in the composition in a proportion ranging from 0.5% to 80% by weight relative to the total weight of the composition.

20. Composition according to Claim 19, **characterized in that** the volatile solvent content ranges from 5% to 80% by weight relative to the total weight of the composition.

21. Composition according to any one of Claims 1 to 20, **characterized in that** it comprises a wax.

22. Composition according to any one of Claims 1 to 21, **characterized in that** it comprises a filler chosen from talc, mica, silica, kaolin, Teflon, starch, natural nacre, boron nitride, microspheres, micro-sponges, polyethylene powders, Nylon powders, silicone resin microbeads and silica microspheres.

23. Composition according to any one of Claims 1 to 22, **characterized in that** the makeup product is a foundation, a makeup rouge, an eyeshadow, an eyeliner, a mascara or a lipstick.

24. Composition according to any one of Claims 1 to 23, **characterized in that** it is in the form of a fluid gel or a stick.

25. Non-therapeutic process for treating the skin and/or the scalp, especially a makeup process, which consists in applying to the skin, mucous membranes and/or the scalp a composition as defined in any one of Claims 1 to 24.

26. Use of an α,ω-substituted oxyalkylenated silicone as defined in any one of Claims 1 to 9 in an anhydrous composition containing no water, which is in the form of a skincare product or a makeup product comprising pigments and a volatile solvent chosen from:
a) linear polydiorganosiloxanes having the general formula below: 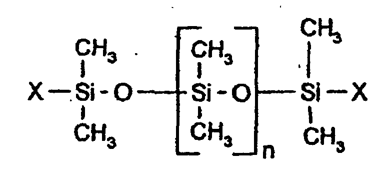 in which:
- X is CH₃ or OH, and
- n is an integer ranging from 0 to 2000;
b) cyclic polydiorganosiloxanes of formula: 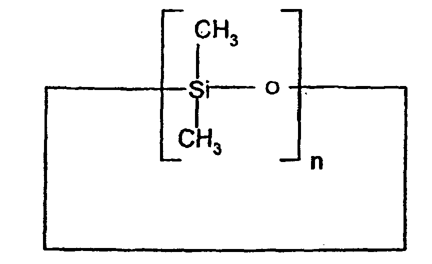 in which:
- n is an integer from 3 to 8;
c) paraffins containing straight or branched hydrocarbon-based chains containing from 8 to 40 carbon atoms;
with the aim of improving the dispersion of the said pigments in the said composition.

27. Use of an α,ω-substituted oxyalkylenated silicone as defined in any one of Claims 1 to 9 in an anhydrous composition containing no water, which is in the form of a skincare product or a makeup product comprising pigments and a volatile solvent chosen from:
a) linear polydiorganosiloxanes having the general formula below: 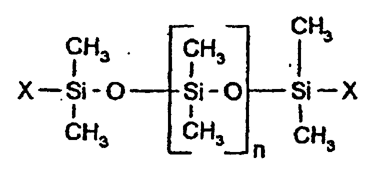 in which:
- X is CH₃ or OH, and
- n is an integer ranging from 0 to 2000;
b) cyclic polydiorganosiloxanes of formula: 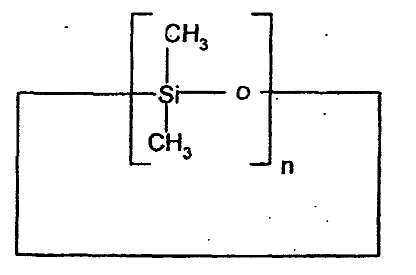 in which:
- n is an integer from 3 to 8;
c) paraffins containing straight or branched hydrocarbon-based chains containing from 8 to 40 carbon atoms;
with the aim of improving the homogeneity of the said composition.

## Patentansprüche

1. Wasserfreie Zusammensetzung, die überhaupt kein Wasser enthält, die in Form eine Hautpflegeprodukts oder eines Schminkprodukts vorliegt, wobei die Zusammensetzung mindestens ein Pigment und mindestens ein flüchtiges Lösemittel enthält, das ausgewählt ist unter
a) den geradkettigen Polydiorganosiloxanen der folgenden allgemeinen Formel 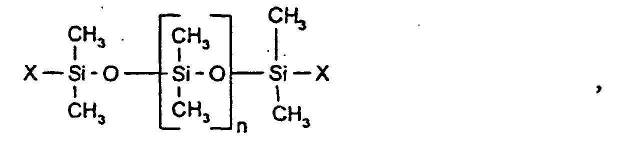 in der bedeuten:
- X CH₃ oder OH und
- n eine ganze Zahl im Bereich von 0 bis 2000;
b) den cyclischen Polydiorganosiloxanen der Formel 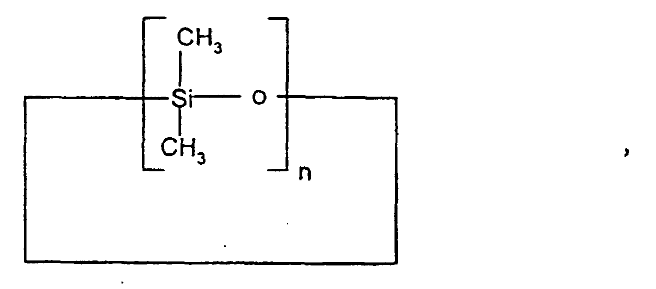 in der bedeutet:
- n eine ganze Zahl im Bereich von 3 bis 8;
c) den Paraffinen mit geradkettigen oder verzweigten Kohlenwasserstoffketten, die 8 bis 40 Kohlenstoffatome aufweisen,
**dadurch gekennzeichnet, dass** sie mindestens ein in α,ω-Stellung substituiertes alkoxyliertes Silicon enthält.

2. Zusammensetzung nach Anspruch 1, **dadurch gekennzeichnet, dass** das in α,ω-Stellung substituierte alkoxylierte Silicon ein siliciumorganisches Polymer mit geradkettiger Struktur ist, das an den beiden Enden der Hauptkette mit Oxyalkylengruppen substituiert ist, die über eine Kohlenwasserstoffgruppe mit den Si-Atomen verbunden sind.

3. Zusammensetzung nach Anspruch 2, **dadurch gekennzeichnet, dass** die Hauptkette keine als Seitengruppen angebundene Oxyalkylengruppen aufweist.

4. Zusammensetzung nach einem der Ansprüche 1 bis 3, **dadurch gekennzeichnet, dass** das in α,ω-Stellung substituierte alkoxylierte Silicon der folgenden allgemeinen Formel (I) 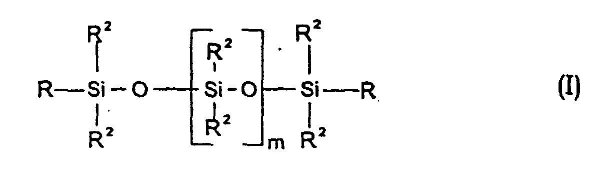 entspricht, in der bedeuten:
- R = -(CH₂)ₚ-O-(C₂H₄O)ₓ (C₃H₆O)_{y}R₁, worin
• R¹ Wasserstoff, CH₃ oder CH₂CH₃ darstellt,
• p eine ganze Zahl im Bereich von 1 bis 5 ist, x im Bereich von 1 bis 100 liegt, y im Bereich von 0 bis 50 liegt,
wobei die (C₂H₄O)- und (C₃H₆O)-Einheiten zufällig oder in Form von Blöcken verteilt sein können,
- die Reste R₂ C₁₋₃-Alkyl oder Phenyl ,
- 5 ≤ m ≤ 300.

5. Zusammensetzung nach Anspruch 4, **dadurch gekennzeichnet, dass** das erfindungsgemäß verwendete, in α,ω-Stellung substituiertes alkoxyliertes Silicon der allgemeinen Formel (I) entspricht, für die alle Reste R² Methylreste sind und
- p im Bereich von 2 bis 4 liegt,
- x im Bereich von 3 bis 100 liegt,
- m im Bereich von 50 bis 200 liegt.

6. Zusammensetzung nach Anspruch 4 oder 5, **dadurch gekennzeichnet, dass** das mittlere Molekulargewicht von R im Bereich von 800 bis 2600 liegt.

7. Zusammensetzung nach einem der Ansprüche 4 bis 6, **dadurch gekennzeichnet, dass** das Gewichtsverhältnis der C₂H₄O-Einheiten zu den C₃H₆O-Einheiten im Bereich von 100:10 bis 20:80 liegt.

8. Zusammensetzung nach Anspruch 7, **dadurch gekennzeichnet, dass** dieses Verhältnis etwa 42/58 beträgt.

9. Zusammensetzung nach einem der Ansprüche 4 bis 8, **dadurch gekennzeichnet, dass** sie das in α,ω-Stellung substituierte alkoxylierte Silicon der folgenden Formel 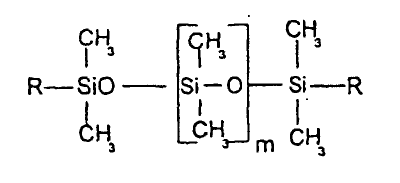 enthält, in der bedeuten:
- m die Zahl 100,
- R die Gruppe (CH₂)₃-O-(C₂H₄O)ₓ(C₃H₆O)_{y}-CH₃, worin x im Bereich von 3 bis 100 liegt, y im Bereich von 1 bis 50 liegt, wobei das Gewichtsverhältnis der Anzahl der C₂H₄O zur Anzahl der C₃H₆O etwa 42/58 beträgt und das mittlere Molekulargewicht von R im Bereich von 800 bis 1500 liegt.

10. Zusammensetzung nach einem der Ansprüche 1 bis 9, **dadurch gekennzeichnet, dass** das in α,ω-Stellung substituierte alkoxylierte Silicon in der Zusammensetzung in einem Anteil enthalten ist, der im Bereich von 0,1 bis 20 Gew.-%, bezogen auf das Gesamtgewicht der Zusammensetzung, liegt.

11. Zusammensetzung nach Anspruch 10, **dadurch gekennzeichnet, dass** der Anteil des in α,ω-Stellung substituierten alkoxylierten Silicons im Bereich von 0,1 bis 10 Gew.-%, bezogen auf das Gesamtgewicht der Zusammensetzung, liegt.

12. Zusammensetzung nach einem der Ansprüche 1 bis 11, **dadurch gekennzeichnet, dass** die Pigmente ausgewählt sind unter: den Dioxiden von Titan, Zirconium und Cer, den Oxiden von Zink, Eisen und Chrom, Eisenblau, den Perlglanzpigmenten, wie Glimmer, der mit Titandioxid, Eisenoxid, natürlichem Pigment oder Bismutoxidchlorid überzogen ist, sowie farbigem Titanglimmer, Ruß, den Lacken von Barium, Strontium, Calcium, Aluminium, den Pigmenten, die mit Siliconverbindungen, wie den PDMS, und/ oder mit Polymeren und insbesondere Polyethylenen und/oder Aminosäuren umhüllt sind.

13. Zusammensetzung nach einem der Ansprüche 1 bis 12, **dadurch gekennzeichnet, dass** die Pigmente in der Zusammensetzung in einem Anteil enthalten sind, der im Bereich von 0,1 bis 20 Gew.-%, bezogen auf das Gesamtgewicht der Zusammensetzung, liegt.

14. Zusammensetzung nach Anspruch 13, **dadurch gekennzeichnet, dass** der Anteil der Pigmente im Bereich von 2 bis 15 Gew.-%, bezogen auf das Gesamtgewicht der Zusammensetzung, liegt.

15. Zusammensetzung nach einem der Ansprüche 1 bis 14, **dadurch gekennzeichnet, dass** das flüchtige Lösemittel eine Viskosität aufweist, die bei 25 °C im Bereich von 0,5 bis 25 Centistokes liegt.

16. Zusammensetzung nach einem der Ansprüche 1 bis 15, **dadurch gekennzeichnet, dass** das flüchtige Lösemittel unter den Paraffinen mit geradkettigen oder verzweigten Kohlenwasserstoffketten, die 10 bis 20 Kohlenstoffatome aufweisen, ausgewählt ist.

17. Zusammensetzung nach einem der Ansprüche 1 bis 16, **dadurch gekennzeichnet, dass** das flüchtige Lösemittel unter Decan, Dodecan, Tetradecan, Tridecan, den C₈₋₂₀-Isoparaffinen ausgewählt ist.

18. Zusammensetzung nach einem der Ansprüche 1 bis 16, **dadurch gekennzeichnet, dass** das flüchtige Lösemittel unter den Paraffinen mit geradkettigen oder verzweigten Kohlenwasserstoffketten ausgewählt ist, die 10 bis 40 Kohlenstoffatome aufweisen und ein Molekulargewicht im Bereich von 160 bis 180 aufweisen oder einen Siedepunkt im Bereich von 105 bis 320 °C aufweisen.

19. Zusammensetzung nach einem der Ansprüche 1 bis 18, **dadurch gekennzeichnet, dass** das flüchtige Lösemittel in der Zusammensetzung in einem Anteil enthalten ist, der im Bereich von 0,5 bis 80 Gew.-%, bezogen auf das Gesamtgewicht der Zusammensetzung, liegt.

20. Zusammensetzung nach Anspruch 19, **dadurch gekennzeichnet, dass** der Anteil des flüchtigen Lösemittels im Bereich von 5 bis 80 Gew.-%, bezogen auf das Gesamtgewicht der Zusammensetzung, liegt.

21. Zusammensetzung nach einem der Ansprüche 1 bis 20, **dadurch gekennzeichnet, dass** sie ein Wachs enthält.

22. Zusammensetzung nach einem der Ansprüche 1 bis 21, **dadurch gekennzeichnet, dass** sie einen Füllstoff enthält, der unter Talk, Glimmer, Kieselsäure, Kaolin, Teflon, Stärke, Perlmutt, Bornitrid, Mikroperlen, Mikroschwämmen, Polyethylenpulvern, Nylonpulvern, Mikrokugeln aus Siliconharz, Mikroperlen aus Kieselsäure ausgewählt ist.

23. Zusammensetzung nach einem der Ansprüche 1 bis 22, **dadurch gekennzeichnet, dass** das Schminkprodukt ein Make-up, ein Rouge, ein Lidschatten, ein Eyeliner, ein Mascara, ein Lippenstift ist.

24. Zusammensetzung nach einem der Ansprüche 1 bis 23, **dadurch gekennzeichnet, dass** sie in Form eines fluiden Gels oder eines Sticks vorliegt.

25. Verfahren zur nicht-therapeutischen Behandlung der Haut und/oder der Kopfhaut, insbesondere Schminkverfahren, das darin besteht, auf die Haut oder die Schleimhäute und/oder auf die Kopfhaut eine wie in einem der Ansprüche 1 bis 24 definierte Zusammensetzung aufzutragen.

26. Verwendung eines in α,ω-Stellung substituierten alkoxylierten Silicons, das wie in einem der Ansprüche 1 bis 9 definiert ist, in einer wasserfreien Zusammensetzung, die kein Wasser enthält, die in Form eine Hautpflegeprodukts oder eines Schminkprodukts vorliegt, die Pigmente und ein flüchtiges Lösemittel enthält, das ausgewählt ist unter
a) den geradkettigen Polydiorganosiloxanen der folgenden allgemeinen Formel 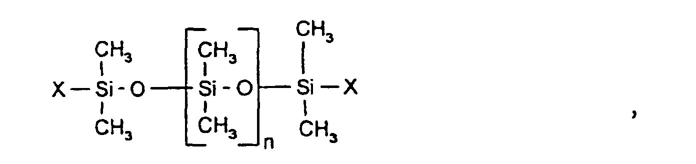 in der bedeuten:
- X CH₃ oder OH und
- n eine ganze Zahl im Bereich von 0 bis 2000;
b) den cyclischen Polydiorganosiloxanen der Formel 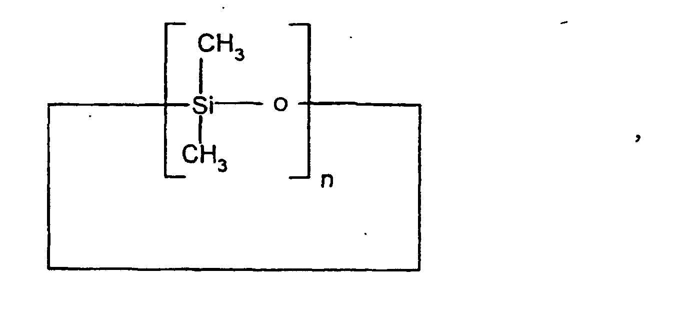 in der bedeutet:
- n eine ganze Zahl im Bereich von 3 bis 8;
c) den Paraffinen mit geradkettigen oder verzweigten Kohlenwasserstoffketten, die 8 bis 40 Kohlenstoffatome aufweisen,
mit dem Ziel, die Verteilung der Pigmente in der Zusammensetzung zu verbessern.

27. Verwendung eines in α,ω-Stellung substituierten alkoxylierten Silicons, das wie in einem der Ansprüche 1 bis 9 definiert ist, in einer wasserfreien Zusammensetzung, die kein Wasser enthält, die in Form eine Hautpflegeprodukts oder eines Schminkprodukts vorliegt, die Pigmente und ein flüchtiges Lösemittel enthält, das ausgewählt ist unter
a) den geradkettigen Polydiorganosiloxanen der folgenden allgemeinen Formel 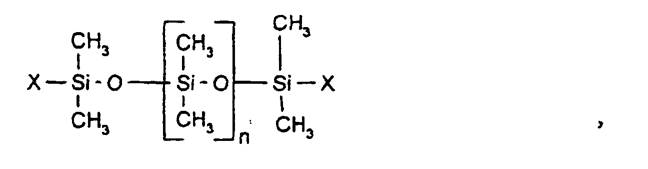 in der bedeuten:
- X CH₃ oder OH und
- n eine ganze Zahl im Bereich von 0 bis 2000;
b) den cyclischen Polydiorganosiloxanen der Formel 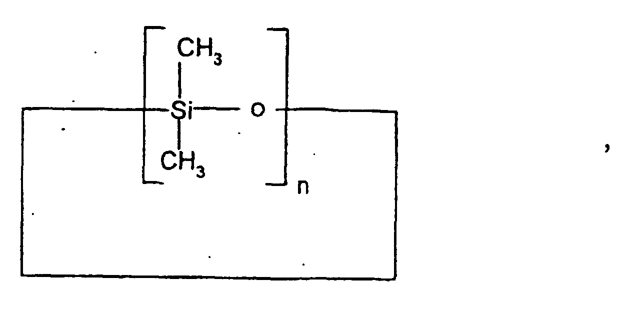 in der bedeutet:
- n eine ganze Zahl im Bereich von 3 bis 8;
c) den Paraffinen mit geradkettigen oder verzweigten Kohlenwasserstoffketten, die 8 bis 40 Kohlenstoffatome aufweisen,
mit dem Ziel, die Homogenität der Zusammensetzung zu verbessern.
